# EUROPEAN PATENT APPLICATION

(11) **EP 4 389 761 A1**
(43) Date of publication of application: **26.06.2024**
(21) Application number: 22857610.4
(22) Date of filing: 05.08.2022
(51) Int. Cl.: C07K 14/165, C12N 15/50, A61K 39/215, A61P 31/14

(54) **POLYPEPTIDE FOR RESISTING NOVEL CORONAVIRUS AND APPLICATION THEREOF**

(30) Priority: 16.08.2021 CN 202110939740
(71) Applicant: Institute Of Microbiology, Chinese Academy Of Sciences, Beijing 100101 (CN)
(72) Inventor: GAO, Fu, Beijing 100101 (CN); WANG, Qihui, Beijing 100101 (CN); WU, Lili, Beijing (CN); ZHENG, Anqi, Beijing 100101 (CN); SUN, Huan, Beijing 100101 (CN); CHAI, Yan, Beijing 100101 (CN); HAN, Pengcheng, Beijing 100101 (CN)
(74) Representative: LLR
(86) International application number: PCT/CN2022/110464
(87) International publication number: WO 2023/020298

(57) **Abstract**

The present application relates to a polypeptide for preventing or treating a novel coronavirus and an application thereof. The polypeptide is P3 polypeptide and any one of P3-1 polypeptide, P3-2 polypeptide, P3-3 polypeptide, P3-4 polypeptide, and P3-5 polypeptide derived from the P3 polypeptide. The amino acid sequences of the P3 polypeptide, the P3-1 polypeptide, the P3-2 polypeptide, the P3-3 polypeptide, the P3-4 polypeptide, and the P3-5 polypeptide are respectively shown in SEQ ID NOs: 1-6. The polypeptide of the present application has a strong inhibitory effect on original strains and a plurality of variant strains of the novel coronavirus, and can be used for preparing a drug or a vaccine for preventing and/or treating diseases caused by the novel coronavirus. The polypeptide is expected to also have the potential of preventing and/or treating new variant strains appearing in the future and sarbecovirus.

## Description

### Cross-Reference

The present application claims the priority of the Chinese patent application entitled "Polypeptide for Resisting Novel Coronavirus and Application Thereof" submitted on August 16, 2021, with the application number of 202110939740.1, and the entire content of which is incorporated in the present application by reference.

### Technical Field

The present application relates to the field of biomedicines, in particular to a polypeptide for preventing or treating the novel coronavirus, a polynucleotide encoding the polypeptide, a nucleic acid construct comprising the polynucleotide, an expression vector and a transformed cell comprising the nucleic acid construct, and a pharmaceutical composition comprising the above, and use thereof in preparing a drug or a vaccine for preventing and/or treating the novel coronavirus.

### Background

The novel coronavirus pneumonia (COVID-19) epidemic has posed a major threat to global public health and human health. SARS-CoV-2 virus is a pathogen of the novel coronavirus pneumonia epidemic, and is the third coronavirus to cause human epidemics since the 21^{st} century. At present, although a variety of vaccines and monoclonal antibody drugs against SARS-CoV-2 have obtained emergency use authorization, most studies have shown that they have a reduced protective effect on the current widely spreading novel coronavirus variant strains such as Alpha (B.1.1.7), Beta (B.1.351) and Gamma (P.1). Therefore, it is urgent to develop broad-spectrum drugs or vaccines against the current epidemic and new variant strains that will appear in the future.

At present, a variety of novel coronaviruse-related coronaviruses (belonging to sarbecovirus) have been reported, such as bat-derived RaTG13, RmYN02, ZC45 and ZXC21, etc., and pangolin-derived GX/P2V/2017 and GD/1/2019. Studies have shown that RaTG13, GX/P2V/2017, GD/1/2019, etc. also have the potential to infect human beings. Therefore, it is urgent to develop drugs or vaccines against these related coronaviruses to deal with possible new epidemics in the future.

### Summary

### Invention Objective

An objective of the present application is to provide a polypeptide for preventing or treating the novel coronavirus, a polynucleotide encoding the polypeptide, a nucleic acid construct comprising the polynucleotide, an expression vector and a transformed cell comprising the nucleic acid construct, and a pharmaceutical composition comprising the above, and use thereof in preparing a drug or a vaccine for preventing or treating the novel coronavirus.

A cvoronavirus spike (S) protein plays an important role in mediating virus invasion process, and has two subunits: S1 and S2, wherein the S1 subunit is responsible for recognizing a receptor; and the S2 subunit mediates the membrane fusion of a viral envelope and a host cell membrane. Heptapeptide repeat sequences HR1 and HR2 in S2 exert the membrane fusion function by forming a six-helix bundle structure. Studies have shown that adding an exogenous HR1 or HR2 polypeptide can inhibit the formation of HR1 and HR2 six-helix bundle structures of the virus itself, thereby inhibiting the membrane fusion process. The polypeptide of the present application is designed based on an HR2 region of the S protein of SARS-CoV-2, which can effectively inhibit the S protein-mediated membrane fusion process, thereby inhibiting the infection of the novel coronavirus.

### Solution

In order to achieve the above objective, the present application provides the following technical solutions.

In a first aspect, the present application provides a polypeptide against novel coronavirus. The polypeptide is any one selected from the group consisting of polypeptide P3 and its derived polypeptides P3-1, P3-2, P3-3, P3-4, and P3-5, wherein
the amino acid sequence of the polypeptide P3 is as follows:
   ISGINASVVNIQKEIDRLNEVAKNLNESLIDLQEL (shown in SEQ ID NO: 1);
the amino acid sequence of the polypeptide P3-1 is as follows:
   ISGINASVVNIQKEIDRLNEVAKNLNESLIDLKEL (shown in SEQ ID NO: 2);
the amino acid sequence of the polypeptide P3-2 is as follows:
   VDLGDISGINASVVNIQKEIDRLNEVAKNLNESLIDLQEL (shown in SEQ ID NO: 3);
the amino acid sequence of the polypeptide P3-3 is as follows:
   VKFGDISGINASVVNIKEEIDRLYEVVKNLNESLIDLQEL (shown in SEQ ID NO: 4);
the amino acid sequence of the polypeptide P3-4 is as follows:
   ISGINASVVNIKEEIDRLNEVAKNLNESLIDLQEL (shown in SEQ ID NO: 5); and
the amino acid sequence of the polypeptide P3-5 is as follows:
   ISGINASVVNIQKEIDRLNEVAKELNESLIDLQEL (shown in SEQ ID NO: 6).

In a preferred specific embodiment, the polypeptide is any one selected from the group consisting of the polypeptide P3-1, the polypeptide P3-2, the polypeptide P3-3, and the polypeptide P3-4.

Preferably, the polypeptide is the polypeptide P3-3.

In another preferred specific embodiment, the polypeptide is linked to a vector protein, such as human serum albumin; the purpose of adding the vector protein includes prolonging the half-life of the polypeptide *in vivo.*

In a second aspect, the present application provides a polynucleotide encoding the polypeptide according to the first aspect.

Preferably, the polynucleotide is DNA or mRNA.

In a specific embodiment, the polynucleotide includes a nucleotide sequence selected from nucleotide sequences shown in SEQ ID NOs: 7-12.

Specifically, the nucleotide sequence encoding the polypeptide P3 is shown in SEQ ID NO: 7; the nucleotide sequence encoding the polypeptide P3-1 is shown in SEQ ID NO: 8; the nucleotide sequence encoding the polypeptide P3-2 is shown in SEQ ID NO: 9; the nucleotide sequence encoding the polypeptide P3-3 is shown in SEQ ID NO: 10; the nucleotide sequence encoding the polypeptide P3-4 is shown in SEQ ID NO: 11; and the nucleotide sequence encoding the polypeptide P3-5 is shown in SEQ ID NO: 12.
SEQ ID NO: 7: 
ATTAGCGGCATTAACGCCTCTGTGGTGAACATTCAGAAGGAGATTGACAGACTGA
ACGAGGTGGCCAAGAACCTGAACGAGTCTCTCATTGACCTGCAGGAGCTG;
SEQ ID NO: 8: 
ATTAGCGGCATTAACGCCTCTGTGGTGAACATTCAGAAGGAGATTGACAGACTGA
ACGAGGTGGCCAAGAACCTGAACGAGTCTCTCATTGACCTGAAGGAGCTG;
SEQ ID NO: 9: 
GTGGACCTGGGCGACATTAGCGGCATTAACGCCTCTGTGGTGAACATTCAGAAGG
AGATTGACAGACTGAACGAGGTGGCCAAGAACCTGAACGAGTCTCTCATTGACCTGC
AGGAGCTG;
SEQ ID NO: 10: 
GTGAAGTTCGGCGACATTAGCGGCATTAACGCCTCTGTGGTGAACATTAAGGAGG
AGATTGACAGACTGTACGAGGTGGTGAAGAACCTGAACGAGTCTCTCATTGACCTGCA
GGAGCTG;
SEQ ID NO: 11: 
ATTAGCGGCATTAACGCCTCTGTGGTGAACATTAAGGAGGAGATTGACAGACTGA
ACGAGGTGGCCAAGAACCTGAACGAGTCTCTCATTGACCTGCAGGAGCTG;
SEQ ID NO: 12:

ATTAGCGGCATTAACGCCTCTGTGGTGAACATTCAGAAGGAGATTGACAGACTGA
ACGAGGTGGCCAAGGAGCTGAACGAGTCTCTCATTGACCTGCAGGAGCTG.

In a third aspect, the present application provides a nucleic acid construct, comprising the polynucleotide according to the second aspect.

Preferably, the nucleic acid construct further comprises at least one expression regulatory element operably linked to the polynucleotide.

In a fourth aspect, the present application provides an expression vector, comprising the nucleic acid construct according to the third aspect.

In a fifth aspect, the present application provides a transformed cell, comprising the polynucleotide according to the second aspect, the nucleic acid construct according to the third aspect, or the expression vector according to the fourth aspect.

In a sixth aspect, the present application provides a pharmaceutical composition, comprising the polypeptide according to the first aspect, the polynucleotide according to the second aspect, the nucleic acid construct according to the third aspect, the expression vector according to the fourth aspect, or the transformed cell according to the fifth aspect, and a pharmaceutically acceptable vehicle and/or excipient.

Preferably, the pharmaceutical composition is in a form of a nasal spray preparation, an oral preparation or a parenteral preparation.

Further preferably, the oral preparation is selected from tablets, capsules, granules, suspensions and pills.

Further preferably, the parenteral preparation is an injectable or bolus-injectable preparation.

Preferably, the pharmaceutical composition is a vaccine composition.

In a seventh aspect, the present application provides use of the polypeptide according to the first aspect, the polynucleotide according to the second aspect, the nucleic acid construct according to the third aspect, the expression vector according to the fourth aspect, or the transformed cell according to the fifth aspect, or the pharmaceutical composition according to the sixth aspect in the preparation of a drug or a vaccine for preventing and/or treating the novel coronavirus infection.

Preferably, the novel coronavirus is a SARS-CoV-2 original strain and/or a SARS-CoV-2 variant and/or sarbecovirus.

Further preferably, the SARS-CoV-2 variant is D614G, Alpha (B.1.1.7), Beta (B.1.351), Gamma (P.1), Kappa (B.1.617.1) and/or Delta (B.1.617.2) strain, and the sarbecovirus is a virus of the sarbecovirus subgenus of β-coronavirus.

In an eighth aspect, the present application provides a method for preventing or treating novel coronavirus, comprising: administering to a subject in need thereof a prophylactically or therapeutically effective amount of the polypeptide according to the first aspect, the polynucleotide according to the second aspect, the nucleic acid construct according to the third aspect, the expression vector according to the fourth aspect, or the transformed cell according to the fifth aspect, or the pharmaceutical composition according to the sixth aspect.

Preferably, the novel coronavirus is the SARS-CoV-2 original strain and/or the SARS-CoV-2 variant and/or the sarbecovirus.

Further preferably, the SARS-CoV-2 variant is the D614G, Alpha (B.1.1.7), Beta (B.1.351), Gamma (P.1), Kappa (B.1.617.1) and/or Delta (B.1.617.2) strain, and the sarbecovirus is a virus of the sarbecovirus subgenus of the β-coronavirus.

### Beneficial Effects

The inventor of the present application designed the polypeptide P3 based on the HR2 region of the S protein of the SARS-CoV-2 virus, and made a series of modifications (including addition or substitution of one or more amino acids) to form derived peptides P3-1, P3-2, P3-3, P3-4 and P3-5. The polypeptide P3 and its derived peptides have strong inhibitory effects on the SARS-CoV-2 original strain and its various variants, and can be used to prepare drugs or vaccines to prevent or treat the novel coronavirus. It is expected that the polypeptides also have the potential to prevent or treat new variant strains that will appear in the future and the sarbecovirus.

### Brief Description of the Drawings

One or more examples are exemplarily described by pictures in corresponding accompanying drawings, and such exemplary description does not constitute a limitation on the examples. The specific term "exemplary" used here means "serving as an instance, example or illustration". Any example described herein as "exemplary" is not necessarily to be construed as advantageous over other examples.

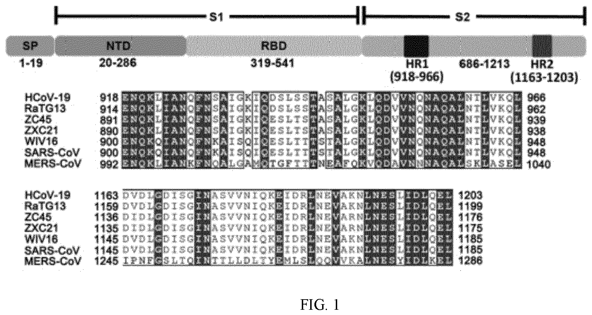

FIG. 1 is a sequence alignment diagram of HR1 and HR2 regions of SARS-CoV-2 and its related coronavirus S protein; and
FIG. 2 shows diagrams demonstrating inhibitory effects of various polypeptides of the present application on pseudoviruses of SARS-CoV-2 original strain and its variants.

### Detailed Description

In order to make objectives, technical solutions, and advantages of examples of the present application clearer, the technical solutions in the examples of the present application are described clearly and completely in the following. Obviously, the described examples are only part of the examples of the present application, not all of them. Based on the examples of the present application, all other examples obtained by those having ordinary skills in the art without making creative efforts shall fall within the scope of protection of the present application. In the entire description and claims, unless otherwise expressly stated, the term "comprise" or the transformation of the term, such as "contain" or "include", will be understood to include the stated elements or components, and not to exclude other elements or components.

In addition, in order to better illustrate the present application, many specific details are given in the following specific description. It is understood to those skilled in the art that the present application can also be implemented without certain specific details. In some examples, the raw materials, elements, methods and means etc., that are well known to those skilled in the art are not described in detail, so as to highlight the gist of the present application.

The following is a detailed description of the present application.

Based on a HR2 region of an S protein of SARS-CoV-2, the present application designed the polypeptide P3 and made a series of modifications (including addition or substitution of one or more amino acids) on the polypeptide P3. The modified polypeptides are named P3-1, P3-2, P3-3, P3-4 and P3-5 respectively. They were directly synthesized by a method commonly used in the art.

### Experimental instruments and materials:

HEK293T cells (purchased from the ATCC Cell Bank).
A pseudovirus packaging skeleton virus G*VSV-delG (purchased from Brain VTA (Wuhan) Co., Ltd).
A eukaryotic expression vector pCAGGS (provided by GENEWIZ, Suzhou).
Hela hACE2 cells (a hACE2 stable transfected cell line constructed by the applicant, see Example 1).
Polypeptide P3, whose amino acid sequence is shown in SEQ ID NO: 1, which is synthesized by Shenzhen Htbio Pharmaceutical Co., Ltd.
Polypeptide P3-1, whose amino acid sequence is shown in SEQ ID NO: 2, which is synthesized by Shenzhen Htbio Pharmaceutical Co., Ltd.
Polypeptide P3-2, whose amino acid sequence is shown in SEQ ID NO: 3, which is synthesized by Shenzhen Htbio Pharmaceutical Co., Ltd.
Polypeptide P3-3, whose amino acid sequence is shown in SEQ ID NO: 4, which is synthesized by Shenzhen Htbio Pharmaceutical Co., Ltd.
Polypeptide P3-4, whose amino acid sequence is shown in SEQ ID NO: 5, which is synthesized by Shenzhen Htbio Pharmaceutical Co., Ltd.
Polypeptide P3-5, whose amino acid sequence is shown in SEQ ID NO: 6, which is synthesized by Shenzhen Htbio Pharmaceutical Co., Ltd.

Sequences of the above six polypeptides are shown in Table 1, in which the bolded parts are the amino acids added or substituted relative to polypeptide P3.

**Table 1. Amino Acid Sequence of Six Polypeptides**

| Name of polypeptide | Amino acid sequence |
|---|---|
| P3 | ISGINASVVNIQKEIDRLNEVAKNLNESLIDLQEL |
| P3-1 | ISGINASVVNIQKEIDRLNEVAKNLNESLIDL**K**EL |
| P3-2 | **VDLGD**ISGINASVVNIQKEIDRLNEVAKNLNESLIDLQEL |
| P3-3 | **VKFGD**ISGINASVVNI**KE**EIDRL**Y**EV**V**KNLNESLIDLQEL |
| P3-4 | ISGINASVVNI**KE**EIDRLNEVAKNLNESLIDLQEL |
| P3-5 | ISGINASVVNIQKEIDRLNEVAK**E**LNESLIDLQEL |

The pseudoviruses of SARS-CoV-2 original strain and its variants of the present application were packaged by the inventors in the laboratory (see Example 2).

### Example 1

### Construction of Hela hACE2 Cell Line

First, the hACE2 gene and a flag tag were fused and expressed; and the sequence was artificially synthesized (this service was provided by GENEWIZ, Suzhou), and then is ligated into the pLVX lentiviral expression vector to obtain the hACE2 expression vector pLVX-hACE2.

The cell line is constructed as follows:
a. Cell preparation: HEK293T cells were plated in a 6-well cell culture plate one day in advance, to make a cell confluence density reach 80-90% on the second day.
b. Transfection: the target plasmids (pLVX-hACE2 and lentiviral packaging plasmids psPAX2 and pMD2.G) were mixed at a ratio of 2: 2: 1; after the plasmid and PEI were uniformly mixed at a ratio of 1: 3, transfection was performed; and after 4-6 hours, the cell culture fluid was replaced with the DMEM medium containing 2% FBS, and continued culture for 48 h.
c. Virus collection and infection: the cell culture supernatant containing packaged lentivirus was filtered with a 0.45 µm sterile filter to remove cell debris, and added to the Hela cells prepared one day in advance.
d. Clone screening: the Hela cells were cultured in the culture medium containing 2 µg/ml puromycin, and positive clones were screened.
e. Establishment of monoclonal cell lines: monoclonal cells were sorted into a 96-well cell culture plate by flow sorting, and then continued to be cultured until single cell clusters were visible under the microscope. After expanding the culture, they were confirmed by target gene sequencing and other methods.

### Example 2

### Packaging of Pseudoviruses of SARS-CoV-2 Original Strain and Variants

### Preparation of expression plasmid of truncated S protein

1) Nucleotides, encoding the last 18 amino acids of the S protein of the SARS-CoV-2 original strain (WT) and its variants (D614G, Alpha (B.1.1.7), Beta (B.1.351), Gamma (P.1), Kappa (B.1.617.1) and Delta (B.1.617.2)), were deleted, and nucleotide sequences of SARS-CoV-2-WT-S-del18, D614G-S-del18, B.1.1.7-S-del18, B.1.351-S-del18, P.1-S-del18, B.1.617.1-S-del18, B.1.617.2-S-del18 (their sequences are shown as SEQ ID NOs: 13-19) were obtained respectively, and synthesized by GENEWIZ, Suzhou.
2) The nucleotide sequences obtained in 1) were cloned into the expression vector pCAGGS to obtain expression plasmids pCAGGS-SARS-CoV-2-WT-S-del18, pCAGGS-D614G-S-del18, pCAGGS-B.1.1.7-S-del18, pCAGGS-B.1.351-S-del18, pCAGGS-P.1-S-del18, pCAGGS-B.1.617.1-S-del18, pCAGGS-B.1.617.2-S-del18 respectively.

### Packaging of Pseudoviruses of SARS-CoV-2 Original Strain and Variants

a. Cell preparation: the HEK293T cells were spread in a 10 cm cell culture dish, to make a cell confluence density reach about 80% on the second day.
b. Transfection: various expression plasmids of each S protein obtained in step 2) were transfected into the cells by PEI by 30 µg plasmids/10 cm cell culture dish; after the target plasmids and the PEI were uniformly mixed at the ratio of 1: 3, transfection was performed; and after 4-6 hours, the culture fluid was replaced (with the DMEM medium containing 10% FBS) and culture at 37°C for 24 h.
c. Virus addition: the pseudovirus packaging skeleton virus G*VSV-delG (purchased from Brain VTA (Wuhan) Co., Ltd) was added to the above transfected HEK293T cells, incubated at 37°C for 2 h; the culture fluid was replaced (with the DMEM medium containing 10% FBS); the VSV-G antibody was added (hybridoma cells expressing the antibody were purchased from the ATCC Cell Bank); and continued to culture in the incubator for 30 h.
d. Virus collection: the supernatant was collected and centrifuged at 3000 rpm for 10 min, and filtered with a 0.45 µm sterile filter in the ultra-clean workbench to remove cell debris; aliquot and frozen in a refrigerator at -80°C.

After the above steps, the pseudoviruses of the SARS-CoV-2 original strain (SARS-CoV-2 WT) and its variants (D614G, Alpha (B.1.1.7), Beta (B.1.351), Gamma (P.1), Kappa (B.1.617.1) and Delta (B.1.617.2)) were obtained respectively.

### Example 3

### Evaluation on Inhibitory Effects of Polypeptides on Pseudoviruses of SARS-CoV-2 Original Strain and Variants Thereof

This example aims to measure the inhibitory effects of the polypeptide P3 and its five derived polypeptides (P3-1, P3-2, P3-3, P3-4 and P3-5) on the pseudoviruses of the original strain (SARS-CoV-2 WT) and the variants (D614G, Alpha (B.1.1.7), Beta (B.1.351), Gamma (P.1), Kappa (B.1.617.1) and Delta (B.1.617.2)).

Experiment groups: Hela hACE2 cells (blank control group, the cells were not infected with the viruses); Hela hACE2 cells + pseudovirus of the original strain or the variant + DMEM medium (negative control group, the cells were infected with the virus, but are not treated with the polypeptide); Hela hACE2 cells + pseudovirus of the original strain or the variant + polypeptide P3 (P3 treatment group); Hela hACE2 cells + pseudovirus of the original strain or the variant + polypeptide P3-1 (P3-1 treatment group); Hela hACE2 cells + pseudovirus of the original strain or the variant + polypeptide P3-3 (P3-3 treatment group); Hela hACE2 cells + pseudovirus of the original strain or the variant + polypeptide P3-4 (P3-4 treatment group); and Hela hACE2 cells + pseudovirus of the original strain or the variant + polypeptide P3-5 (P3-5 treatment group).

Preparation of polypeptide stock solutions: Using DMSO, the polypeptides P3, P3-1, P3-2, P3-3, P3-4, and P3-5 were respectively prepared into 10 mM mother liquors, and then the mother liquors were diluted into 20 µM using the DMEM medium containing 10% FBS as the stock solutions for further gradient dilution.

Preparation of polypeptide gradient dilutions: the above each of 20 µM polypeptide stock solutions was diluted by 2 folds using the DMEM medium containing 10% FBS, and diluted into a total of 9 gradient dilutions (10 µM, 5 µM, 2.5 µM, 1.25 µM, 0.625 µM, 0.3125 µM, 0.156 µM, 0.078 µM, and 0.039 µM respectively) with 3 duplicate wells for each gradient, and 50 µL in each well.

Determination of the dosage of the pseudoviruses: the SARS-CoV-2 original strain or variant pseudovirus stock solution and a series of dilutions were quantified on the Hela hACE2 cells, and the dilution when 1000 FFU appears will be used as the virus dosage to evaluate the inhibitory effect of the polypeptide (the dilution fold was between 6 times and 20 times).

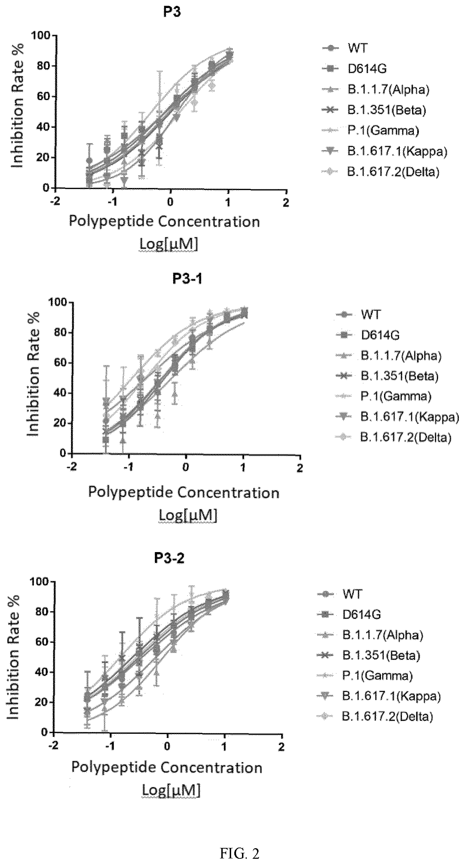

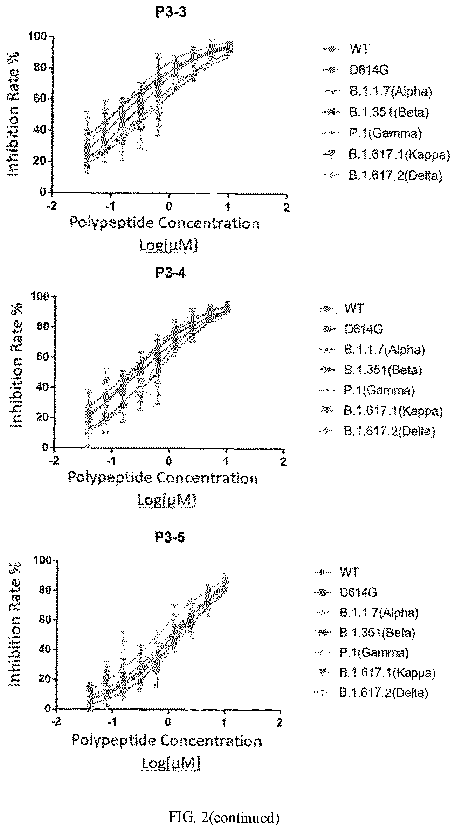

The determination method for the virus inhibitory effect is as follows:
a. Hela hACE2 cells were plated in the 96-well cell culture plate and culture until the cell confluence density reach 80-90% on the second day.
b. According to the above experiment groups, the above gradient dilutions of each polypeptide were taken respectively, and an equal volume (50 µL) of quantitative pseudovirus dilution was added for uniform mixing; and then, incubation was performed at 37°C for 1 h.
c. The supernatant on the 96-well cell culture plate in step a was carefully discarded; the polypeptide-pseudovirus mixture (100 µL/well) in step b was added, and continued to be cultured in the incubator for 15-24 h.
d. The number of infected cells were counted by a high-content microscope, and inhibition rates of each polypeptide at different concentrations were calculated; then IC₅₀ of each polypeptide was calculated by GraphPad (as shown in FIG. 2), and results were shown in Table 2; and the fold increases in the inhibitory effects of the derived polypeptides P3-1 to P3-5 on the pseudovirus of SARS-CoV-2 original strain compared to the polypeptide P3 were calculated, and results were shown in Table 3.

**Table 2. Inhibitory Effects of Polypeptides on Pseudoviruses of SARS-CoV-2 Original Strain and Variants(IC₅₀)**

| IC₅₀ (µM) | WT | D614G | B.1.1.7 (Alpha) | B.1.351 (Beta) | P.1 (Gamma) | B.1.617.1 (Kappa) | B.1.617.2 (Delta) |
|---|---|---|---|---|---|---|---|
| p3 | 0.78 | 0.76 | 0.89 | 0.96 | 0.45 | 1.25 | 1.39 |
| P3-1 | 0.37 | 0.41 | 0.54 | 0.36 | 0.11 | 0.19 | 0.19 |
| P3-2 | 0.34 | 0.30 | 0.80 | 0.23 | 0.14 | 0.60 | 0.27 |
| P3-3 | 0.23 | 0.16 | 0.37 | 0.10 | 0.11 | 0.43 | 0.32 |
| P3-4 | 0.24 | 0.30 | 0.59 | 0.20 | 0.22 | 0.48 | 0.55 |
| P3-5 | 1.59 | 1.47 | 1.43 | 1.24 | 0.63 | 1.06 | 1.82 |

**Table 3. Fold Increases in the Inhibitory Effects of the Derived Polypeptides P3-1 - P3-5 on the Pseudovirus of SARS-CoV-2 Original Strain Compared to the Polypeptide P3**

| Increase multiple | WT | D614G | B.1.1.7 (Alpha) | B.1.351 (Beta) | P.1 (Gamma) | B.1.617.1 (Kappa) | B.1.617.2 (Delta) |
|---|---|---|---|---|---|---|---|
| p3 | 1.0 | 1.0 | 0.9 | 0.8 | 1.8 | 0.6 | 0.6 |
| P3-1 | 2.1 | 1.9 | 1.5 | 2.2 | 7.1 | 4.0 | 4.2 |
| P3-2 | 2.3 | 2.6 | 1.0 | 3.4 | 5.5 | 1.3 | 2.9 |
| P3-3 | 3.4 | 5.0 | 2.1 | 7.5 | 7.2 | 1.8 | 2.4 |
| P3-4 | 3.3 | 2.6 | 1.3 | 4.0 | 3.6 | 1.6 | 1.4 |
| P3-5 | 0.5 | 0.5 | 0.5 | 0.6 | 1.3 | 0.7 | 0.4 |

It can be seen from Table 2 and Table 3 that the polypeptide P3 has a good inhibitory effect on the SARS-CoV-2 original strain and its various variants (0.45 µM ≤ IC50 ≤ 1.39 µM). Compared with the polypeptide P3, the inhibitory effects of the derived polypeptides P3-1, P3-2, P3-3 and P3-4 on the SARS-CoV-2 original strain and its various variants are improved to varying degrees, especially the inhibitory effect of the polypeptide P3-3 is the most obviously improved. Although the inhibitory effect of P3-5 decreased slightly, the inhibitory effect of P3-5 on the Kappa variant strain was slightly higher than that of P3.

It can be seen from Table 2 and Table 3 that the polypeptides of the present application can be used to prevent or treat diseases caused by the novel coronavirus; and it is speculated that the polypeptides may also be widely used for diseases caused by sarbecovirus.

In the process of SARS-CoV-2 invading host cells, the polypeptides of the present application inhibit virus invasion by inhibiting the S protein-mediated membrane fusion process, and thus are suitable for treating or preventing the original stain and its variants (such as D614G, B.1.1.7 (Alpha), B.1.351 (Beta), P.1 (Gamma), Kappa (B.1.617.1) or Delta (B.1.617.2)) of the novel coronavirus. It is speculated that the polypeptides also have the inhibitory effects on sarbecovirus and new variant strains of the novel coronavirus that will appear in the future, and have potential wide application values.

Finally, it is to be noted that: the above examples are only used to explain the technical solutions of the present application and shall not be construed as limitation. Although the present application has been described in detail with respect to the previously described examples, those having ordinary skills in the art should understand that the technical solutions recorded in the examples can still be modified, or part of its technical features may be substituted with equivalents; and such modifications or substitutions do not deviate the nature of the technical solutions from the spirit and scope of the technical solutions of the various examples in the present application.

### Industrial Applicability

The present application provides a polypeptide for preventing or treating novel coronavirus and its application. The polypeptide is any one selected from the group consisting of polypeptide P3 and its derived polypeptides P3-1, P3-2, P3-3, P3-4, and P3-5. The polypeptide provided by the present application has a strong inhibitory effect on SARS-CoV-2 original strain and its various variants, and can be used for preparing a drug or a vaccine for the prevention and/or treatment of diseases caused by the novel coronavirus. It is expected that the polypeptide has also a potential for preventing and/or treating new variant strains that will appear in the future and sarbecovirus, and will have great clinical application prospects and values.

## Claims

1. A polypeptide against novel coronavirus, which is any one selected from the group consisting of polypeptide P3 and its derived polypeptides P3-1, P3-2, P3-3, P3-4, and P3-5, wherein
an amino acid sequence of the polypeptide P3 is shown as SEQ ID NO: 1;
an amino acid sequence of the polypeptide P3-1 is shown as SEQ ID NO: 2;
an amino acid sequence of the polypeptide P3-2 is shown as SEQ ID NO: 3;
an amino acid sequence of the polypeptide P3-3 is shown as SEQ ID NO: 4;
an amino acid sequence of the polypeptide P3-4 is shown as SEQ ID NO: 5; and
an amino acid sequence of the polypeptide P3-5 is shown as SEQ ID NO: 6.

2. The polypeptide according to claim 1, wherein the polypeptide is any one selected from the group consisting of the polypeptide P3-1, the polypeptide P3-2, the polypeptide P3-3, and the polypeptide P3-4.

3. The polypeptide against novel coronavirus according to claim 1 or 2, wherein the polypeptide is linked to a vector protein, preferably, the vector protein is human serum albumin.

4. A polynucleotide encoding the polypeptide according to any one of claims 1-3.

5. The polynucleotide according to claim 4, wherein the polynucleotide is DNA or mRNA;
preferably, the polynucleotide comprises a nucleotide sequence selected from nucleotide sequences shown as SEQ ID NO: 7-12.

6. A nucleic acid construct, comprising the polynucleotide according to claim 4 or 5.

7. The nucleic acid construct according to claim 6, further comprising at least one expression regulatory element operably linked to the polynucleotide.

8. An expression vector comprising the nucleic acid construct according to claim 6 or 7.

9. A transformed cell comprising the polynucleotide according to claim 4 or 5, the nucleic acid construct according to claim 6 or 7, or the expression vector according to claim 8.

10. A pharmaceutical composition comprising the polypeptide according to any one of claims 1-3, the polynucleotide according to claim 4 or 5, the nucleic acid construct according to claim 6 or 7, the expression vector according to claim 8, or the transformed cell according to claim 9, and a pharmaceutically acceptable vehicle and/or excipient.

11. The pharmaceutical composition according to claim 10, wherein the pharmaceutical composition is in a form of a nasal spray preparation, an oral preparation, or a parenteral preparation;
preferably, the oral preparation is selected from tablets, capsules, granules, suspensions, and pills; and preferably, the parenteral preparation is an injectable or bolus-injectable preparation;
preferably, the pharmaceutical composition is a vaccine composition.

12. Use of the polypeptide according to any one of claims 1-3, the polynucleotide according to claim 4 or 5, the nucleic acid construct according to claim 6 or 7, the expression vector according to claim 8, the transformed cell according to claim 9, or the pharmaceutical composition according to claim 10 or 11 in the preparation of a drug or a vaccine for preventing and/or treating the novel coronavirus.

13. The use according to claim 12, wherein the novel coronavirus is a SARS-CoV-2 original strain and/or a SARS-CoV-2 variant and/or sarbecovirus;
preferably, the SARS-CoV-2 variant is D614G, Alpha (B.1.1.7), Beta (B.1.351), Gamma (P.1), Kappa (B.1.617.1) and/or Delta (B.1.617.2) strain, and the sarbecovirus is a virus of sarbecovirus subgenus of β-coronavirus.

14. A method for preventing or treating novel coronavirus, comprising: administering to a subject in need thereof a prophylactically or therapeutically effective amount of the polypeptide according to any one of claims 1-3, the polynucleotide according to claim 4 or 5, the nucleic acid construct according to claim 6 or 7, the expression vector according to claim 8, the transformed cell according to claim 9, or the pharmaceutical composition according to claim 10 or 11.

15. The method according to claim 14, wherein the novel coronavirus is a SARS-CoV-2 original strain and/or a SARS-CoV-2 variant and/or sarbecovirus;
preferably, the SARS-CoV-2 variant is D614G, Alpha (B.1.1.7), Beta (B.1.351), Gamma (P.1), Kappa (B.1.617.1) and/or Delta (B.1.617.2) strain, and the sarbecovirus is a virus of sarbecovirus subgenus of β-coronavirus.
